(19) Europäisches Patentamt
European Patent Office
Office européen des brevets

(11) EP 1 234 044 B1

(12) **EUROPÄISCHE PATENTSCHRIFT**

(45) Veröffentlichungstag und Bekanntmachung des Hinweises auf die Patenterteilung:
**29.10.2008 Patentblatt 2008/44**

(21) Anmeldenummer: **00990613.2**

(22) Anmeldetag: **24.11.2000**

(51) Int Cl.:
*C12N 15/62* (2006.01) *C07K 14/16* (2006.01)

(86) Internationale Anmeldenummer:
**PCT/EP2000/011733**

(87) Internationale Veröffentlichungsnummer:
**WO 2001/037881 (31.05.2001 Gazette 2001/22)**

(54) **MEMBRANVERANKERTE GP41-FUSIONSPEPTIDE UND DEREN VERWENDUNGEN**

GENE THERAPY OF HIV-POSITIVE PATIENTS BY THE EXPRESSION OF MEMBRANE-ANCHORED GP41 PEPTIDES

THERAPIE GENETIQUE DE MALADES SEROPOSITIFS POUR LE VIH PAR L'EXPRESSION DE PEPTIDES GP41 A ANCRAGE PAR MEMBRANE

(84) Benannte Vertragsstaaten:
**AT BE CH CY DE DK ES FI FR GB GR IE IT LI LU MC NL PT SE TR**
Benannte Erstreckungsstaaten:
**LT LV MK RO SI**

(30) Priorität: **25.11.1999 DE 19957838**

(43) Veröffentlichungstag der Anmeldung:
**28.08.2002 Patentblatt 2002/35**

(73) Patentinhaber: **Heinrich-Pette-Institut
20251 Hamburg (DE)**

(72) Erfinder: **VON LAER, Meike-Dorothee
22529 Hamburg (DE)**

(74) Vertreter: **UEXKÜLL & STOLBERG
Patentanwälte
Beselerstrasse 4
22607 Hamburg (DE)**

(56) Entgegenhaltungen:
**WO-A-00/45833** **FR-A- 2 762 851**
**US-A- 5 858 744**

- **MUNOZ-BARROSO ISABEL ET AL: "Role of the membrane-proximal domain in the initial stages of human immunodeficiency virus type 1 envelope glycoprotein-mediated membrane fusion." JOURNAL OF VIROLOGY, Bd. 73, Nr. 7, Juli 1999 (1999-07), Seiten 6089-6092, XP002170871 ISSN: 0022-538X**
- **HILDINGER M ET AL: "FMEV vectors: Both retroviral long terminal repeat and leader are important for high expression in transduced hematopoietic cells." GENE THERAPY, Bd. 5, Nr. 11, November 1998 (1998-11), Seiten 1575-1579, XP001007102 ISSN: 0969-7128**
- **JOSHI SANGEETA BAGAI ET AL: "A core trimer of the paramyxovirus fusion protein: Parallels to influenza virus hemagglutinin and HIV-1 gp41." VIROLOGY, Bd. 248, Nr. 1, 15. August 1998 (1998-08-15), Seiten 20-34, XP002170872 ISSN: 0042-6822**
- **D C CHAN ET AL: "Evidence that a prominent cavity in the coiled coil of HIV type 1 gp41 is an attractive drug target" PROCEEDINGS OF THE NATIONAL ACADEMY OF SCIENCES OF USA, NATIONAL ACADEMY OF SCIENCE. WASHINGTON,US, Bd. 95, Dezember 1998 (1998-12), Seiten 15613-15617, XP002126805 ISSN: 0027-8424**
- **SALZWEDEL KARL ET AL: "A conserved tryptophan-rich motif in the membrane-proximal region of the human immunodeficiency virus type 1 gp41 ectodomain is important for Env-mediated fusion and virus infectivity." JOURNAL OF VIROLOGY, Bd. 73, Nr. 3, März 1999 (1999-03), Seiten 2469-2480, XP002170873 ISSN: 0022-538X**
- **HILDINGER M ET AL: "BICISTRONIC RETROVIRAL VECTORS FOR COMBINING MYELOPROTECTION WITH CELL-SURFACE MARKING" GENE THERAPY,MACMILLAN PRESS LTD., BASINGSTOKE,GB, Bd. 6, Nr. 7, 1999, Seiten 1222-1230, XP000990645 ISSN: 0969-7128**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

• KILBY J M ET AL: "POTENT SUPPRESSION OF HIV-1 REPLICATION IN HUMANS BY T-20, A PEPTIDE INHIBITOR OF GP41-MEDIATED VIRUS ENTRY" NATURE MEDICINE,US,NATURE PUBLISHING, CO, Bd. 4, 1998, Seiten 1302-1307, XP000909990 ISSN: 1078-8956

**SEQUENZPROTOKOLL**

<110> Heinrich-Pette-Institut

<120> Gentherapie von HIV-Infizierten durch Expression von Membran-verankerten gp41-Peptiden

<130> p055363

<140>
<141>

<160> 15

<170> PatentIn Ver. 2.0

<210> 1
<211> 4148
<212> DNA
<213> Kuenstliche Sequenz

<220>
<223> Beschreibung der küenstlichen Sequenz: M87 (STHM) - Retroviraler Vektor MPIN, der 5' von der IRES-NEO-Kassette ein Insert enthaelt, das fuer Membran-verankertes T-20-Peptid kodiert

<220>
<221> misc_feature
<222> (1438)..(1773)
<223> STHM (Vektor-Insert, das den fuer das Membran-verankerte T-20-Peptid kodierenden Bereich enthaelt)

<220>
<221> CDS
<222> (1528)..(1773)
<223> Bereich im M87 (STHM)-Insert, der fuer Membran-verankertes T-20-Fusionsprotein einschliesslich Signalpeptid kodiert

<220>
<221> sig_peptide
<222> (1438)..(1527)

<220>
<221> CDS
<222> (1528)..(1773)

<223> T-20-Fusionsprotein mit Membrananker

<220>
<221> misc feature
<222> (1528)..(1635)
<223> Fuer T-20 kodierender Bereich

<220>
<221> misc_feature
<222> (1636)..(1683)
<223> Fuer Hinge kodierender Bereich

<220>
<221> misc_feature
<222> (1684)..(1773)
<223> Fuer Membrananker kodierender Bereich

<220>
<221> misc_feature
<222> (1793)..(2421)
<223> Polio-IRES

<220>
<221> misc_feature
<222> (2380)..(3267)
<223> neoR cDNA

<220>
<221> misc_feature
<222> (31)..(36)
<223> NheI-Schnittstelle

<220>
<221> misc_feature
<222> (370)..(377)
<223> AscI-Schnittstelle

<220>
<221> misc_feature
<222> (400)..(405)
<223> XmaI-Schnittstelle

<220>
<221> misc_feature
<222> (696)..(701)
<223> SacII-Schnittstelle

<220>
<221> misc_feature
<222> (1421)..(1426)
<223> SacII-Schnittstelle

<220>
<221> misc_feature
<222> (1429)..(1436)
<223> NotI-Schnittstelle

<220>

<221> misc_feature
<222> (1775)..(1780)
<223> SalI-Schnittstelle

<220>
<221> misc_feature
<222> (1787)..(1792)
<223> SphI-Schnittstelle

<220>
<221> misc_feature
<222> (1793)..(1798)
<223> HindIII-Schnittstelle

<220>
<221> misc_feature
<222> (2422)..(2429)
<223> NotI-Schnittstelle

<220>
<221> misc_feature
<222> (3002)..(3007)
<223> SphI-Schnittstelle

<220>
<221> misc_feature
<222> (3447)..(3452)
<223> HindIII-Schnittstelle

<220>
<221> misc_feature
<222> (3586)..(3591)
<223> NheI-Schnittstelle

<220>
<221> misc_feature
<222> (4000)..(4007)
<223> AscI-Schnittstelle

<220>
<221> misc_feature
<222> (4030)..(4035)
<223> XmaI-Schnittstelle

<400> 1

```
aatgaaagac cccacctgta ggtttggcaa gctagcttaa gtaacgccat tttgcaaggc 60
atggaaaata cataactgag aatagagaag ttcagatcaa ggttaggaac agagagacag 120
cagaatatgg gccaaacagg atatctgtgg taagcagttc ctgccccgct cagggccaag 180
aacagatggt ccccagatgc ggtcccgccc tcagcagttt ctagagaacc atcagatgtt 240
tccagggtgc cccaaggacc tgaaaatgac cctgtgcctt atttgaacta accaatcagt 300
tcgcttctcg cttctgttcg cgcgcttctg ctccccgagc tcaataaaag agcccacaac 360
ccctcactcg gcgcgccagt cctccgattg actgagtcgc ccgggtaccc gtgttctcaa 420
taaaccctct tgcagttgca tccgactcgt ggtctcgctg atccttggga gggtctcctc 480
agattgattg actgcccacc tcgggggtct ttcatttgga ggttccaccg agatttggag 540
acccctgccc agggaccacc gacccccccg ccgggaggta agctggccag cggtcgtttc 600
gtgtctgtct ctgtctttgt gcgtgtttgt gccggcatct aatgtttgcg cctgcgtctg 660
tactagttgg ctaactagat ctgtatctgg cggtcccgcg gaagaactga cgagttcgta 720
ttcccggccg cagcccctgg gagacgtccc agcggcctcg ggggcccgtt ttgtggccca 780
ttctgtatca gttaacctac ccgagtcgga ctttttggag ctccgccact gtccgagggg 840
tacgtggctt tgttgggga cgagagacag agacacttcc cgcccccgtc tgaatttttg 900
ctttcggttt tacgccgaaa ccgcgccgcg cgtcttgtct gctgcagcat cgttctgtgt 960
tgtctctgtc tgactgtgtt tctgtatttg tctgaaaatt agggccagac tgttaccact 1020
cccttaagtt tgaccttagg tcactggaaa gatgtcgagc ggatcgctca caaccagtcg 1080
gtagatgtca agaagagacg ttgggttacc ttctgctctg cagaatggcc aacctttaac 1140
gtcggatggc cgcgagacgg cacctttaac cgagacctca tcacccaggt taagatcaag 1200
gtcttttcac ctggcccgca tggacaccca gaccaggtcc cctacatcgt gacctgggaa 1260
gccttggctt ttgacccccc tccctgggtc aagccctttg tacaccctaa gcctccgcct 1320
```

```
cctcttcctc catccgcccc gtctctcccc cttgaacctc ctcgttcgac cccgcctcga 1380

tcctcccttt atccagccct cactccttct ctaggcgcca ccgcggtggc ggccgcc    1437

atg ggg gca ggt gcc acc ggc cgc gcc atg gac ggg ccg cgc ctg ctg   1485
Met Gly Ala Gly Ala Thr Gly Arg Ala Met Asp Gly Pro Arg Leu Leu
 1               5                  10                  15

ctg ttg ctg ctt ctg ggg gtg tcc ctt gga ggt gcc aga tct tac act   1533
Leu Leu Leu Leu Leu Gly Val Ser Leu Gly Gly Ala Arg Ser Tyr Thr
            20                  25                  30

agc tta ata cac tcc tta att gaa gaa tcg caa aac cag caa gaa aag   1581
Ser Leu Ile His Ser Leu Ile Glu Glu Ser Gln Asn Gln Gln Glu Lys
        35                  40                  45

aat gaa caa gaa tta ttg gaa tta gat aaa tgg gca agt ttg tgg aat   1629
Asn Glu Gln Glu Leu Leu Glu Leu Asp Lys Trp Ala Ser Leu Trp Asn
    50                  55                  60

tgg ttt aga tct gtt cca aga gac tgt gga tgc aaa ccc tgt ata tgt   1677
Trp Phe Arg Ser Val Pro Arg Asp Cys Gly Cys Lys Pro Cys Ile Cys
65                  70                  75                  80

acc ctc atc cct gtc tat tgc tcc atc ctg gct gct gtg gtt gtg ggc   1725
Thr Leu Ile Pro Val Tyr Cys Ser Ile Leu Ala Ala Val Val Val Gly
                85                  90                  95

ctt gtg gcc tac ata gcc ttc aag agg tgg aac agg ggg atc ctc tag   1773
Leu Val Ala Tyr Ile Ala Phe Lys Arg Trp Asn Arg Gly Ile Leu
            100                 105                 110

agtcgacctg caggcatgca agcttaaaac agctctgggg ttgtacccac cccagaggcc 1833

cacgtggcgg ctagtactcc ggtattgcgg tacccttgta cgcctgtttt atactccctt 1893

cccgtaactt agacgcacaa aaccaagttc aatagaaggg ggtacaaacc agtaccacca 1953

cgaacaagca cttctgtttc cccggtgatg tcgtatacac tgcttgcgtg gttgaaagcg 2013

acggatccgt tatccgctta tgtacttcga gaagcccagt accacctcgg aatctcaatg 2073

cgttgcgctc agcactcaac cccatagtgt acttaggctg atgagtctcc acatccctca 2133

ccggtgacgg tggtccaggt tgcgttggcg gcctacctat ggctaacgcc atgggacgct 2193

agttgtgaac aaggtgtgaa gagcctattg agctacataa gaatcctccg gcccctgaat 2253

gcggctaatc ccaaccctcg gagcaggtgg tcacaaacca gtgattggcc tgtcgtaacg 2313

cgaagtccgt ggcggaaccg actactttgg gtgtccgtgt ttccttttat tttattgtgg 2373

ctgcttatgg tgacaatcac agattgttat cataaagcga attggattgc ggccgctcta 2433

gaactagtgg atctaattcc tgcagccaat atgggatcgg ccattgaaca agatggattg 2493

cacgcaggtt ctccggccgc ttgggtggag aggctattcg gctatgactg ggcacaacag 2553

acaatcggct gctctgatgc cgccgtgttc cggctgtcag cgcaggggcg cccggttctt 2613

tttgtcaaga ccgacctgtc cggtgccctg aatgaactgc aggacgaggc agcgcggcta 2673

tcgtggctgg ccacgacggg cgttccttgc gcagctgtgc tcgacgttgt cactgaagcg 2733
```

ggaagggact ggctgctatt gggcgaagtg ccggggcagg atctcctgtc atctcacctt 2793
gctcctgccg agaaagtatc catcatggct gatgcaatgc ggcggctgca tacgcttgat 2853
ccggctacct gcccattcga ccaccaagcg aaacatcgca tcgagcgagc acgtactcgg 2913
atggaagccg gtcttgtcga tcaggatgat ctggacgaag agcatcaggg gctcgcgcca 2973
gccgaactgt tcgccaggct caaggcgcgc atgcccgacg gcgaggatct cgtcgtgacc 3033
catggcgatg cctgcttgcc gaatatcatg gtggaaaatg gccgcttttc tggattcatc 3093
gactgtggcc ggctgggtgt ggcggaccgc tatcaggaca tagcgttggc tacccgtgat 3153
attgctgaag agcttggcgg cgaatgggct gaccgcttcc tcgtgcttta cggtatcgcc 3213
gctcccgatt cgcagcgcat cgccttctat cgccttcttg acgagttctt ctgagcggga 3273
ctctggggtt cgaaatgacc gaccaagcga cgcccaacct gccatcacga gatttcgatt 3333
ccaccgccgc cttctatgaa aggttgggct tcggaatcgt tttccgggac gccggctgga 3393
tgatcctcca gcgcggggat ctcatgctgg agttcttcgc ccaccccgga tccaagctta 3453
tcgataggcc taggcctatc gataggccta ggcctatcga taggcctaac acgagccata 3513
gatagaataa aagattttat ttagtctcca gaaaaagggg ggaatgaaag accccacctg 3573
taggtttggc aagctagctt aagtaagcca ttttgcaagg catggaaaaa tacataactg 3633
agaatagaga agttcagatc aaggttagga acagagagac aggagaatat gggccaaaca 3693
ggatatctgt ggtaagcagt tcctgccccg gctcagggcc aagaacagtt ggaacagcag 3753
aatatgggcc aaacaggata tctgtggtaa gcagttcctg ccccggctca gggccaagaa 3813
cagatggtcc ccagatgcgg tcccgccctc agcagtttct agagaaccat cagatgtttc 3873
cagggtgccc caaggacctg aaatgaccct gtgccttatt tgaactaacc aatcagttcg 3933
cttctcgctt ctgttcgcgc gcttctgctc cccgagctca ataaaagagc ccacaacccc 3993
tcactcggcg cgccagtcct ccgatagact gcgtcgcccg ggtacccgt attcccaata 4053
aagcctcttg ctgtttgcat ccgaatcgtg gactcgctga tccttgggag ggtctcctca 4113
gattgattga ctgcccacct cgggggtctt tcatt 4148

<210> 2
<211> 111
<212> PRT
<213> Artificial Sequence

<400> 2

```
Met Gly Ala Gly Ala Thr Gly Arg Ala Met Asp Gly Pro Arg Leu Leu
1               5                   10                  15

Leu Leu Leu Leu Leu Gly Val Ser Leu Gly Gly Ala Arg Ser Tyr Thr
            20                  25                  30

Ser Leu Ile His Ser Leu Ile Glu Glu Ser Gln Asn Gln Gln Glu Lys
        35                  40                  45

Asn Glu Gln Glu Leu Leu Glu Leu Asp Lys Trp Ala Ser Leu Trp Asn
    50                  55                  60

Trp Phe Arg Ser Val Pro Arg Asp Cys Gly Cys Lys Pro Cys Ile Cys
65                  70                  75                  80

Thr Leu Ile Pro Val Tyr Cys Ser Ile Leu Ala Ala Val Val Val Gly
                85                  90                  95

Leu Val Ala Tyr Ile Ala Phe Lys Arg Trp Asn Arg Gly Ile Leu
            100                 105                 110
```

<210> 3
<211> 9709
<212> DNA
<213> Human immunodeficiency virus type 1

<220>
<221> CDS
<222> (1)..(2562)
<223> Fuer Huell-Glykoprotein von HIV-1 kodierender Bereich

<400> 3

```
atg aga gtg aag gag aag tat cag cac ttg tgg aga tgg ggg tgg aaa      48
Met Arg Val Lys Glu Lys Tyr Gln His Leu Trp Arg Trp Gly Trp Lys
  1               5                  10                  15

tgg ggc acc atg ctc ctt ggg ata ttg atg atc tgt agt gct aca gaa      96
Trp Gly Thr Met Leu Leu Gly Ile Leu Met Ile Cys Ser Ala Thr Glu
            20                  25                  30

aaa ttg tgg gtc aca gtc tat tat ggg gta cct gtg tgg aag gaa gca     144
Lys Leu Trp Val Thr Val Tyr Tyr Gly Val Pro Val Trp Lys Glu Ala
        35                  40                  45

acc acc act cta ttt tgt gca tca gat gct aaa gca tat gat aca gag     192
Thr Thr Thr Leu Phe Cys Ala Ser Asp Ala Lys Ala Tyr Asp Thr Glu
    50                  55                  60

gta cat aat gtt tgg gcc aca cat gcc tgt gta ccc aca gac ccc aac     240
Val His Asn Val Trp Ala Thr His Ala Cys Val Pro Thr Asp Pro Asn
 65                  70                  75                  80

cca caa gaa gta gta ttg gta aat gtg aca gaa aat ttt aac atg tgg     288
Pro Gln Glu Val Val Leu Val Asn Val Thr Glu Asn Phe Asn Met Trp
                 85                  90                  95

aaa aat gac atg gta gaa cag atg cat gag gat ata atc agt tta tgg     336
Lys Asn Asp Met Val Glu Gln Met His Glu Asp Ile Ile Ser Leu Trp
            100                 105                 110

gat caa agc cta aag cca tgt gta aaa tta acc cca ctc tgt gtt agt     384
Asp Gln Ser Leu Lys Pro Cys Val Lys Leu Thr Pro Leu Cys Val Ser
        115                 120                 125

tta aag tgc act gat ttg aag aat gat act aat acc aat agt agt agc     432
Leu Lys Cys Thr Asp Leu Lys Asn Asp Thr Asn Thr Asn Ser Ser Ser
    130                 135                 140

ggg aga atg ata atg gag aaa gga gag ata aaa aac tgc tct ttc aat     480
Gly Arg Met Ile Met Glu Lys Gly Glu Ile Lys Asn Cys Ser Phe Asn
145                 150                 155                 160
```

```
atc agc aca agc ata aga gat aag gtg cag aaa gaa tat gca ttc ttt    528
Ile Ser Thr Ser Ile Arg Asp Lys Val Gln Lys Glu Tyr Ala Phe Phe
                165                 170                 175

tat aaa ctt gat ata gta cca ata gat aat acc agc tat agg ttg ata    576
Tyr Lys Leu Asp Ile Val Pro Ile Asp Asn Thr Ser Tyr Arg Leu Ile
            180                 185                 190

agt tgt aac acc tca gtc att aca cag gcc tgt cca aag gta tcc ttt    624
Ser Cys Asn Thr Ser Val Ile Thr Gln Ala Cys Pro Lys Val Ser Phe
        195                 200                 205

gag cca att ccc ata cat tat tgt gcc ccg gct ggt ttt gcg att cta    672
Glu Pro Ile Pro Ile His Tyr Cys Ala Pro Ala Gly Phe Ala Ile Leu
    210                 215                 220

aaa tgt aat aat aag acg ttc aat gga aca gga cca tgt aca aat gtc    720
Lys Cys Asn Asn Lys Thr Phe Asn Gly Thr Gly Pro Cys Thr Asn Val
225                 230                 235                 240

agc aca gta caa tgt aca cat gga atc agg cca gta gta tca act caa    768
Ser Thr Val Gln Cys Thr His Gly Ile Arg Pro Val Val Ser Thr Gln
                245                 250                 255

ctg ctg tta aat ggc agt cta gca gaa gaa gat gta gta att aga tct    816
Leu Leu Leu Asn Gly Ser Leu Ala Glu Glu Asp Val Val Ile Arg Ser
            260                 265                 270

gcc aat ttc aca gac aat gct aaa acc ata ata gta cag ctg aac aca    864
Ala Asn Phe Thr Asp Asn Ala Lys Thr Ile Ile Val Gln Leu Asn Thr
        275                 280                 285

tct gta gaa att aat tgt aca aga ccc aac aac aat aca aga aaa agt    912
Ser Val Glu Ile Asn Cys Thr Arg Pro Asn Asn Asn Thr Arg Lys Ser
    290                 295                 300

atc cgt atc cag agg gga cca ggg aga gca ttt gtt aca ata gga aaa    960
Ile Arg Ile Gln Arg Gly Pro Gly Arg Ala Phe Val Thr Ile Gly Lys
305                 310                 315                 320

ata gga aat atg aga caa gca cat tgt aac att agt aga gca aaa tgg    1008
Ile Gly Asn Met Arg Gln Ala His Cys Asn Ile Ser Arg Ala Lys Trp
                325                 330                 335

aat gcc act tta aaa cag ata gct agc aaa tta aga gaa caa ttt gga    1056
Asn Ala Thr Leu Lys Gln Ile Ala Ser Lys Leu Arg Glu Gln Phe Gly
            340                 345                 350

aat aat aaa aca ata atc ttt aag caa tcc tca gga ggg gac cca gaa    1104
Asn Asn Lys Thr Ile Ile Phe Lys Gln Ser Ser Gly Gly Asp Pro Glu
        355                 360                 365

att gta acg cac agt ttt aat tgt gga ggg gaa ttt ttc tac tgt aat    1152
Ile Val Thr His Ser Phe Asn Cys Gly Gly Glu Phe Phe Tyr Cys Asn
    370                 375                 380

tca aca caa ctg ttt aat agt act tgg ttt aat agt act tgg agt act    1200
Ser Thr Gln Leu Phe Asn Ser Thr Trp Phe Asn Ser Thr Trp Ser Thr
385                 390                 395                 400

gaa ggg tca aat aac act gaa gga agt gac aca atc aca ctc cca tgc    1248
Glu Gly Ser Asn Asn Thr Glu Gly Ser Asp Thr Ile Thr Leu Pro Cys
                405                 410                 415
```

```
aga ata aaa caa ttt ata aac atg tgg cag gaa gta gga aaa gca atg      1296
Arg Ile Lys Gln Phe Ile Asn Met Trp Gln Glu Val Gly Lys Ala Met
            420                 425                 430

tat gcc cct ccc atc agt gga caa att aga tgt tca tca aat att act      1344
Tyr Ala Pro Pro Ile Ser Gly Gln Ile Arg Cys Ser Ser Asn Ile Thr
        435                 440                 445

ggg ctg cta tta aca aga gat ggt ggt aat aac aac aat ggg tcc gag      1392
Gly Leu Leu Leu Thr Arg Asp Gly Gly Asn Asn Asn Asn Gly Ser Glu
    450                 455                 460

atc ttc aga cct gga gga ggc gat atg agg gac aat tgg aga agt gaa      1440
Ile Phe Arg Pro Gly Gly Gly Asp Met Arg Asp Asn Trp Arg Ser Glu
465                 470                 475                 480

tta tat aaa tat aaa gta gta aaa att gaa cca tta gga gta gca ccc      1488
Leu Tyr Lys Tyr Lys Val Val Lys Ile Glu Pro Leu Gly Val Ala Pro
                485                 490                 495

acc aag gca aag aga aga gtg gtg cag aga gaa aaa aga gca gtg gga      1536
Thr Lys Ala Lys Arg Arg Val Val Gln Arg Glu Lys Arg Ala Val Gly
            500                 505                 510

ata gga gct ttg ttc ctt ggg ttc ttg gga gca gca gga agc act atg      1584
Ile Gly Ala Leu Phe Leu Gly Phe Leu Gly Ala Ala Gly Ser Thr Met
        515                 520                 525

ggc tgc acg tca atg acg ctg acg gta cag gcc aga caa tta ttg tct      1632
Gly Cys Thr Ser Met Thr Leu Thr Val Gln Ala Arg Gln Leu Leu Ser
    530                 535                 540

gat ata gtg cag cag cag aac aat ttg ctg agg gct att gag gcg caa      1680
Asp Ile Val Gln Gln Gln Asn Asn Leu Leu Arg Ala Ile Glu Ala Gln
545                 550                 555                 560

cag cat ctg ttg caa ctc aca gtc tgg ggc atc aaa cag ctc cag gca      1728
Gln His Leu Leu Gln Leu Thr Val Trp Gly Ile Lys Gln Leu Gln Ala
                565                 570                 575

aga atc ctg gct gtg gaa aga tac cta aag gat caa cag ctc ctg ggg      1776
Arg Ile Leu Ala Val Glu Arg Tyr Leu Lys Asp Gln Gln Leu Leu Gly
            580                 585                 590

att tgg ggt tgc tct gga aaa ctc att tgc acc act gct gtg cct tgg      1824
Ile Trp Gly Cys Ser Gly Lys Leu Ile Cys Thr Thr Ala Val Pro Trp
        595                 600                 605

aat gct agt tgg agt aat aaa tct ctg gaa cag att tgg aat aac atg      1872
Asn Ala Ser Trp Ser Asn Lys Ser Leu Glu Gln Ile Trp Asn Asn Met
    610                 615                 620

acc tgg atg gag tgg gac aga gaa att aac aat tac aca agc tta ata      1920
Thr Trp Met Glu Trp Asp Arg Glu Ile Asn Asn Tyr Thr Ser Leu Ile
625                 630                 635                 640

cac tcc tta att gaa gaa tcg caa aac cag caa gaa aag aat gaa caa      1968
His Ser Leu Ile Glu Glu Ser Gln Asn Gln Gln Glu Lys Asn Glu Gln
                645                 650                 655

gaa tta ttg gaa tta gat aaa tgg gca agt ttg tgg aat tgg ttt aac      2016
Glu Leu Leu Glu Leu Asp Lys Trp Ala Ser Leu Trp Asn Trp Phe Asn
            660                 665                 670
```

```
ata aca aat tgg ctg tgg tat ata aaa tta ttc ata atg ata gta gga      2064
Ile Thr Asn Trp Leu Trp Tyr Ile Lys Leu Phe Ile Met Ile Val Gly
        675                 680                 685

ggc ttg gta ggt tta aga ata gtt ttt gct gta ctt tct ata gtg aat      2112
Gly Leu Val Gly Leu Arg Ile Val Phe Ala Val Leu Ser Ile Val Asn
    690                 695                 700

aga gtt agg cag gga tat tca cca tta tcg ttt cag acc cac ctc cca      2160
Arg Val Arg Gln Gly Tyr Ser Pro Leu Ser Phe Gln Thr His Leu Pro
705                 710                 715                 720

atc ccg agg gga ccc gac agg ccc gaa gga ata gaa gaa gaa ggt gga      2208
Ile Pro Arg Gly Pro Asp Arg Pro Glu Gly Ile Glu Glu Glu Gly Gly
                725                 730                 735

gag aga gac aga gac aga tcc att cga tta gtg aac gga tcc tta gca      2256
Glu Arg Asp Arg Asp Arg Ser Ile Arg Leu Val Asn Gly Ser Leu Ala
            740                 745                 750

ctt atc tgg gac gat ctg cgg agc ctg tgc ctc ttc agc tac cac cgc      2304
Leu Ile Trp Asp Asp Leu Arg Ser Leu Cys Leu Phe Ser Tyr His Arg
        755                 760                 765

ttg aga gac tta ctc ttg att gta acg agg att gtg gaa ctt ctg gga      2352
Leu Arg Asp Leu Leu Leu Ile Val Thr Arg Ile Val Glu Leu Leu Gly
    770                 775                 780

cgc agg ggg tgg gaa gcc ctc aaa tat tgg tgg aat ctc cta cag tat      2400
Arg Arg Gly Trp Glu Ala Leu Lys Tyr Trp Trp Asn Leu Leu Gln Tyr
785                 790                 795                 800

tgg agt cag gaa cta aag aat agt gct gtt aac ttg ctc aat gcc aca      2448
Trp Ser Gln Glu Leu Lys Asn Ser Ala Val Asn Leu Leu Asn Ala Thr
                805                 810                 815

gcc ata gca gta gct gag ggg aca gat agg gtt ata gaa gta tta caa      2496
Ala Ile Ala Val Ala Glu Gly Thr Asp Arg Val Ile Glu Val Leu Gln
            820                 825                 830

gca gct tat aga gct att cgc cac ata cct aga aga ata aga cag ggc      2544
Ala Ala Tyr Arg Ala Ile Arg His Ile Pro Arg Arg Ile Arg Gln Gly
        835                 840                 845

ttg gaa agg att ttg cta                                              2562
Leu Glu Arg Ile Leu Leu
    850
```

<210> 4
<211> 854
<212> PRT
<213> Human immunodeficiency virus type 1

<400> 4

```
Met Arg Val Lys Glu Lys Tyr Gln His Leu Trp Arg Trp Gly Trp Lys
 1               5                  10                  15

Trp Gly Thr Met Leu Leu Gly Ile Leu Met Ile Cys Ser Ala Thr Glu
            20                  25                  30

Lys Leu Trp Val Thr Val Tyr Tyr Gly Val Pro Val Trp Lys Glu Ala
        35                  40                  45
```

Thr Thr Thr Leu Phe Cys Ala Ser Asp Ala Lys Ala Tyr Asp Thr Glu
50 55 60

Val His Asn Val Trp Ala Thr His Ala Cys Val Pro Thr Asp Pro Asn
65 70 75 80

Pro Gln Glu Val Val Leu Val Asn Val Thr Glu Asn Phe Asn Met Trp
85 90 95

Lys Asn Asp Met Val Glu Gln Met His Glu Asp Ile Ile Ser Leu Trp
100 105 110

Asp Gln Ser Leu Lys Pro Cys Val Lys Leu Thr Pro Leu Cys Val Ser
115 120 125

Leu Lys Cys Thr Asp Leu Lys Asn Asp Thr Asn Thr Asn Ser Ser Ser
130 135 140

Gly Arg Met Ile Met Glu Lys Gly Glu Ile Lys Asn Cys Ser Phe Asn
145 150 155 160

Ile Ser Thr Ser Ile Arg Asp Lys Val Gln Lys Glu Tyr Ala Phe Phe
165 170 175

Tyr Lys Leu Asp Ile Val Pro Ile Asp Asn Thr Ser Tyr Arg Leu Ile
180 185 190

Ser Cys Asn Thr Ser Val Ile Thr Gln Ala Cys Pro Lys Val Ser Phe
195 200 205

Glu Pro Ile Pro Ile His Tyr Cys Ala Pro Ala Gly Phe Ala Ile Leu
210 215 220

Lys Cys Asn Asn Lys Thr Phe Asn Gly Thr Gly Pro Cys Thr Asn Val
225 230 235 240

Ser Thr Val Gln Cys Thr His Gly Ile Arg Pro Val Val Ser Thr Gln
245 250 255

Leu Leu Leu Asn Gly Ser Leu Ala Glu Glu Asp Val Val Ile Arg Ser
260 265 270

Ala Asn Phe Thr Asp Asn Ala Lys Thr Ile Ile Val Gln Leu Asn Thr
275 280 285

Ser Val Glu Ile Asn Cys Thr Arg Pro Asn Asn Asn Thr Arg Lys Ser
290 295 300

Ile Arg Ile Gln Arg Gly Pro Gly Arg Ala Phe Val Thr Ile Gly Lys
305 310 315 320

Ile Gly Asn Met Arg Gln Ala His Cys Asn Ile Ser Arg Ala Lys Trp
325 330 335

Asn Ala Thr Leu Lys Gln Ile Ala Ser Lys Leu Arg Glu Gln Phe Gly
340 345 350

Asn Asn Lys Thr Ile Ile Phe Lys Gln Ser Ser Gly Gly Asp Pro Glu
355 360 365

Ile Val Thr His Ser Phe Asn Cys Gly Gly Glu Phe Phe Tyr Cys Asn
370 375 380

Ser Thr Gln Leu Phe Asn Ser Thr Trp Phe Asn Ser Thr Trp Ser Thr
385 390 395 400

Glu Gly Ser Asn Asn Thr Glu Gly Ser Asp Thr Ile Thr Leu Pro Cys
405 410 415

Arg Ile Lys Gln Phe Ile Asn Met Trp Gln Glu Val Gly Lys Ala Met
420 425 430

Tyr Ala Pro Pro Ile Ser Gly Gln Ile Arg Cys Ser Ser Asn Ile Thr
435 440 445

Gly Leu Leu Leu Thr Arg Asp Gly Gly Asn Asn Asn Asn Gly Ser Glu
450 455 460

Ile Phe Arg Pro Gly Gly Gly Asp Met Arg Asp Asn Trp Arg Ser Glu
465 470 475 480

Leu Tyr Lys Tyr Lys Val Val Lys Ile Glu Pro Leu Gly Val Ala Pro
485 490 495

Thr Lys Ala Lys Arg Arg Val Val Gln Arg Glu Lys Arg Ala Val Gly
500 505 510

Ile Gly Ala Leu Phe Leu Gly Phe Leu Gly Ala Ala Gly Ser Thr Met
515 520 525

Gly Cys Thr Ser Met Thr Leu Thr Val Gln Ala Arg Gln Leu Leu Ser
530 535 540

Asp Ile Val Gln Gln Gln Asn Asn Leu Leu Arg Ala Ile Glu Ala Gln
545 550 555 560

Gln His Leu Leu Gln Leu Thr Val Trp Gly Ile Lys Gln Leu Gln Ala
565 570 575

Arg Ile Leu Ala Val Glu Arg Tyr Leu Lys Asp Gln Gln Leu Leu Gly
580 585 590

Ile Trp Gly Cys Ser Gly Lys Leu Ile Cys Thr Thr Ala Val Pro Trp
595 600 605

Asn Ala Ser Trp Ser Asn Lys Ser Leu Glu Gln Ile Trp Asn Asn Met
610 615 620

Thr Trp Met Glu Trp Asp Arg Glu Ile Asn Asn Tyr Thr Ser Leu Ile
625 630 635 640

His Ser Leu Ile Glu Glu Ser Gln Asn Gln Gln Glu Lys Asn Glu Gln
645 650 655

Glu Leu Leu Glu Leu Asp Lys Trp Ala Ser Leu Trp Asn Trp Phe Asn
660 665 670

Ile Thr Asn Trp Leu Trp Tyr Ile Lys Leu Phe Ile Met Ile Val Gly
675 680 685

Gly Leu Val Gly Leu Arg Ile Val Phe Ala Val Leu Ser Ile Val Asn
690 695 700

Arg Val Arg Gln Gly Tyr Ser Pro Leu Ser Phe Gln Thr His Leu Pro
705 710 715 720

```
Ile Pro Arg Gly Pro Asp Arg Pro Glu Gly Ile Glu Glu Glu Gly Gly
                725                 730                 735

Glu Arg Asp Arg Asp Arg Ser Ile Arg Leu Val Asn Gly Ser Leu Ala
            740                 745                 750

Leu Ile Trp Asp Asp Leu Arg Ser Leu Cys Leu Phe Ser Tyr His Arg
        755                 760                 765

Leu Arg Asp Leu Leu Leu Ile Val Thr Arg Ile Val Glu Leu Leu Gly
    770                 775                 780

Arg Arg Gly Trp Glu Ala Leu Lys Tyr Trp Trp Asn Leu Leu Gln Tyr
785                 790                 795                 800

Trp Ser Gln Glu Leu Lys Asn Ser Ala Val Asn Leu Leu Asn Ala Thr
                805                 810                 815

Ala Ile Ala Val Ala Glu Gly Thr Asp Arg Val Ile Glu Val Leu Gln
            820                 825                 830

Ala Ala Tyr Arg Ala Ile Arg His Ile Pro Arg Arg Ile Arg Gln Gly
        835                 840                 845

Leu Glu Arg Ile Leu Leu
    850
```

<210> 5
<211> 30
<212> DNA
<213> Kuenstliche Sequenz

<220>
<223> Beschreibung der kuenstlichen Sequenz: Primer SPNot+

<400> 5
gcggccgcca tgggggcagg tgccaccggc 30

<210> 6
<211> 30
<212> DNA
<213> Kuenstliche Sequenz

<220>
<223> Beschreibung der kuenstlichen Sequenz: Primer SPBg12-

<400> 6
agatctggca cctccaaggg acaccccag 30

<210> 7
<211> 30
<212> DNA
<213> Kuenstliche Sequenz

<220>
<223> Beschreibung der kuenstlichen Sequenz: Primer T20Bgl+

<400> 7
agatcttaca ctagcttaat acactcctta 30

<210> 8
<211> 39
<212> DNA
<213> Kuenstliche Sequenz

<220>
<223> Beschreibung der kuenstlichen Sequenz: Primer T20Bgl-RGD+

<400> 8
agatctagag gcgactacac tagcttaata cactcctta 39

<210> 9
<211> 33
<212> DNA
<213> Kuenstliche Sequenz

<220>
<223> Beschreibung der kuenstlichen Sequenz: Primer T20Hind-

<400> 9
aagcttatta aaaccaattc cacaaacttg ccc 33

<210> 10
<211> 54
<212> DNA
<213> Kuenstliche Sequenz

<220>
<223> Beschreibung der kuenstlichen Sequenz: Primer hingeTMBgl+

<400> 10
agatctgttc caagagactg tggatgcaaa ccctgtatat gtaccctcat ccct 54

<210> 11
<211> 30
<212> DNA
<213> Kuenstliche Sequenz

<220>
<223> Beschreibung der kuenstlichen Sequenz: Primer U3-

<400> 11
cgcgcgaaca gaagcgagaa g 21

<210> 12
<211> 20
<212> DNA
<213> Kuenstliche Sequenz

<220>
<223> Beschreibung der kuenstlichen Sequenz: Primer T20Bgl-

<400> 12
agatctaaac caattccaca aacttgccc 29

<210> 13
<211> 29
<212> DNA

<213> Kuenstliche Sequenz

<220>
<223> Beschreibung der kuenstlichen Sequenz: Primer RGD-T20Not+

<400> 13
aaagcggccg ccatgagggg cgattacact agcttaatac actccttaat tg 52

<210> 14
<211> 25
<212> DNA
<213> Kuenstliche Sequenz

<220>
<223> Beschreibung der kuenstlichen Sequenz: Primer T20Not+

<400> 14
aaagcggccg ccatgtacac tagcttaata cactccttaa ttg 43

<210> 15
<211> 22
<212> DNA
<213> Kuenstliche Sequenz

<220>
<223> Beschreibung der kuenstlichen Sequenz: Primer T20mtsHind-

<400> 15
aagcttacta gggtgcggca agaagaacag ggagaagaac ggctgcaaac caattccaca 60

## Beschreibung

Die vorliegende Erfindung betrifft die gentherapeutische Behandlung einer HIV-Infektion durch Expression Membran-verankerter gp41-Peptide. Dazu werden erstmals Vektoren zur Verfügung gestellt, die für ein Fusionsprotein kodieren, das ein aus gp41 von HIV abgeleitetes Peptid und einen carboxyterminal durch einen flexiblen Linker angehängten Transmembrananker enthält.

Zur Therapie von HIV-Infektionen wurden bereits die unterschiedlichsten Therapieansätze vorgeschlagen. Es hat sich jedoch gezeigt, daß die verfügbaren Wirkstoffe für viele Patienten nicht verträglich sind. Um die Therapie von AIDS zu verbessern, wird ständig nach neuen Angriffspunkten und Wirkstoffen mit unterschiedlichem Toxizitätsprofil gesucht. In diesem Zusammenhang wurden bereits verschiedene gentherapeutische Ansätze vorgeschlagen, um die unterschiedlichen Schritte bei der Replikation von HIV zu hemmen (vergl. Sorg T. & Methali, M. Transfus. Sci. 18, 277-289 (1997)).

Von Wild et al. (Wild, C.T. Shugars, D.C. Greenwell, T.K. McDanal, C.B. & Matthews, T.J. Proc. Natl.Acad. Sci U.S.A. 91, 9770-9774 (1994)) wurde ein Therapieansatz vorgeschlagen, der auf der Beobachtung beruht, daß Peptide, die von dem Transmembranprotein gp41 (vgl. SEQ ID NO: 4 entsprechen numerische Kennzahl <400> gemäß WIPO Standard ST. 25: 4) des HIV abgeleitet sind, wie z.B. das früher als DP178 bezeichnete Peptid T-20, wirksam die HIV-Fusion und den Eintritt in die Zelle verhindern können. Bei T-20 handelt es sich um ein Peptid, das mit dem C-terminalen Heptad repeat, einem von zwei Sequenzabschnitten (vgl. Positionen 539-589 und 622-662 der SEQ ID NO: 4) in Regionen der Ektodomäne des gp41, überlappt, und die HIV-Infektion in vitro effektiv inhibieren kann (Weissenhborn, W., Dessen, A., Harrison, S.C. Skehel, J.J. & Wiley, D.C. Nature 387, 426-430 (1997); Chan, D.C. Fass, D., Berger, J.M. & Kim, P.S. Cell 89. 263-273 (1997); Furuta, R.A., Wild, C.T., Weng Y & Weiss, C.D. Nat. Struct. Biol. 5, 276-279 (1998)).

Im Rahmen einer klinischen Studie (Kilby, J.M., Hopkins, S., Venetta, T.M. et al. Nat.Med. 4, 1302-1307 (1998)) erwies sich T-20 bei kurzfristiger Verabreichung als sicheres Mittel, das in der Lage ist, die HIV-Replikation wirksam zu inhibieren. Da jedoch sehr große Mengen des Peptids benötigt werden, um einen antiviralen Effekt zu erzielen und die Peptide bei oraler Gabe nicht bioverfügbar sind, eine sehr kurze Halbwertszeit besitzen und die Herstellung in großem Maßstab noch immer extrem kostenaufwendig ist, liegt der Erfindung die Aufgabe zugrunde, die intrazelluläre Immunisierung mit Peptiden, die von gp41 abgeleitet sind, durch einen gentherapeutischen Ansatz zu ermöglichen.

Von den Erfindern wurden daher zunächst die für T-20 kodierenden Sequenzen 5' zur IRES-NEO-Cassette (IRES: Internal ribosomal entry site; NEO: Neomycin-Resistenzgen) des retroviralen Vektors MPIN kloniert (vergl. Figur 1, Hildinger et al., Hum. Gene Ther. 9 (1998) 33-42). Um die Sekretion des T-20 zu erreichen, wurde das Peptid einerseits direkt hinter dem Signalpeptid des humanen low affinity nerve growth factor receptor (LNGFR, Fehse et al., Human Gene Therapy 8 (1997) 1815) exprimiert (Konstrukte M85 und M86), zum anderen wurde ein Konstrukt gewählt, das die kodierenden Sequenzen für ein von Kaposi fibroblast growth factor abgeleitetes Membran-Translokationssignal (mts) (Aminosäurepositionen 43-58 im mts-Protein; Rojas, M., Donahue, J.P., Tan, Z., Lin, Y.Z. Nat.Biotechnol. 16, 370-375 (1998)) in Leserichtung (in frame) mit T20 enthielt (vergl. Konstrukte M89 und M90 in Figur 1). Mit Hilfe dieser Konstrukte wurden retrovirale Vektoren durch Transfektion von Phoenix-Verpackungszellen (Grignani, F., Kinsella, T., Mencarelli, A. et al., Cancer Res. 58, 14-19 (1998)) hergestellt, und die Überstände wurden verwendet, um die T-Helferzellinie PM-1 (Bou-Habib, D.C. et al., J. Virol. 68 6006-6013 (1994)) zu infizieren. Als Kontrolle wurde der MPIN-Vektor verwendet, der ausschließlich das Neomycin-Resistenzgen als Fremdgen enthielt. Nach G418-Selektion wurden die Massenkulturen mit HIV-1 infiziert, das aus den proviralen Klonen NL4-3 (Adachi, A., Gendelman H.E., Koenig, S., Folks, T., Willey, R., Rabson, A. & Martin, M.A., J. Virol. 59 284-291 (1986)) oder NL4-3/GFP (Welker, R., Harris, M., Cardel, B. & Krausslich, H.G. J.Virol. 72, 8833-8840 (1998)) gewonnen wurde. Diese beiden Klone unterscheiden sich dadurch, daß bei NL4-3/GFP das Green fluorescent protein (GFP) anstelle des nef-Proteins exprimiert wird, so daß die HIV-1-Replikation anhand der p24-Antigen-Produktion bzw. mittels Durchflußzytometrie überwacht werden kann. Entgegen allen Erwartungen führte die Expression von T-20-Peptid unter Verwendung der o.g. retroviralen Vektorkonstrukte aber überraschenderweise zu keinerlei antiviralen Aktivität.

In diesen Konstrukten wurde daher zusätzlich eine für das Integrin-bindende RGD-Peptid kodierende Sequenz in frame mit der für T-20 kodierenden Region einkloniert. Der Herstellung dieser Konstrukte lag die Überlegung zugrunde, daß das RGD-Motiv die sekretierten Peptide an der Zellmembran festhalten könnte. Aber auch mit dem RGD-enthaltenden sekretierten T-20 Peptid (vergl. Figur 1, M86) wurde noch eine reproduzierbare HIV-Replikation beobachtet.

Im Rahmen der vorliegenden Erfindung wurde nun überraschenderweise festgestellt, daß sich die Produktion von p24 und die Verbreitung von NL4-3/GFP sehr stark vermindern läßt, wenn man ein Fusionsprotein exprimiert, das zusätzlich zu einem aminoterminalen gp41-Peptid daran carboxyterminal über einen flexiblen Linker angehängten Transmembrananker enthält. Unter "gp41-Peptid" wird in diesem Zusammenhang ein Fragment des gp41-Proteins des HIV oder ein Fragment, eine Variante oder Mutante desselben verstanden.

So wurde beispielsweise festgestellt, daß sich die p24-Produktion um mehr als 2 Zehnerpotenzen vermindern läßt, wenn man PM-1 mit einem retroviralen Vektor transduziert, der ein Fusionsprotein exprimiert, bei dem T-20 C-terminal über einen flexiblen Peptid-Linker mit einem Transmembran-Peptid (membrane spanning domain, MSD) verknüpft ist (vergl. Figur 1, M87), wobei das Fusionsprotein die in SEQ ID NO: 2 angegebene Sequenz aufweist.

Gegenstand der vorliegenden Erfindung ist daher eine Nukleinsäuresequenz der allgemeinen Formel

$$5'- SP - FI - Hinge - MSD -3',$$

in der

| | |
|---|---|
| "5'" | das 5'-Ende der Nukleinsäuresequenz bezeichnet, |
| "3'" | das 3'-Ende der Nukleinsäuresequenz bezeichnet, |
| "SP" | für ein Signalpeptid kodiert, das den Transfer eines exprimierten Peptids in das endoplasmatische Reticulum ermöglicht, |
| "FI" | für ein Fragment des gp41-Proteins von HIV (vorzugs- weise HIV-1) kodiert, das einen Abschnitt aus einer Heptad Repeat-Region enthält, wobei der Abschnitt eine Sequenz von mindestens 28 Aminosäuren zwischen Aminosäurepositionen 539-589 und 622-662 in SEQ ID NO:4 oder Aminosäurepositionen 31-66 in SEQ ID NO:2 aufweist,<br>oder für eine Aminosäuresequenz kodiert, die von der Sequenz dieses Fragments durch Austausch, Deletion oder Insertion einzelner Aminosäuren abweicht, |
| "MSD" | für einen Transmembrananker eines Typ I-Membranproteins kodiert und |
| "Hinge" | für eine Proteinsequenz kodiert, die als flexibler Linker die von "FI" und "MSD" kodierten Peptide ver- bindet. |

Die für das Signalpeptid kodierende Sequenz ist von humanen, nicht-immunogenen Proteinen abgeleitet, vorzugsweise ausgewählt aus der Gruppe bestehend aus Sequenzen, die für Signalpeptide zellulärer Membranproteine kodieren, wie zum Beispiel des (humanen) low affinity nerve growth factor receptor (LNGFR), des Interleukin-2-Rezeptors (IL-2R) und des granulocyte macrophage colony stimulating factor receptor (GM-CSFR).

Als Transmembrananker eines Typ I-Membranproteins (d.h. eines Membranproteins, dessen N-Terminus sich außerhalb und dessen C-terminus sich in der Zelle befindet), dienen Proteine, deren zytoplasmatische Domäne deletiert sein sollte, um eventuell unerwünschte Signaltransduktion durch das Protein zu vermeiden. In Voruntersuchungen läßt sich abgeklären, daß von den exprimierten Bereichen keine Signaltransduktionswirkungen ausgehen und sie nicht mit anderen, ähnlichen Membranproteinen oligomerisieren und auf diese Weise eine indirekte Wirkung auf die Zellfunktionen ausüben. Es kommen vorzugsweise Peptide aus der Gruppe bestehend aus der Transmembranregion des LNGFR oder des CD34 in Frage. Die Nukleinsäuresequenz enthält daher vorzugsweise als MSD eine für diese Transmembrananker bzw. für die Fragmente mit deletierter zytoplasmatischer Domäne kodierende Nukleinsäuresequenz.

Als flexible Linker können erfindungsgemäß sämtliche flexiblen Peptide dienen, die eine flexible Verbindung zwischen dem gp41-Peptid und dem Transmembrananker ermöglichen, wie z.B. der Hinge von Immunglobulin G (IgG), der Linker des humanen P-Glykoproteins (C.A. Hrycyna et al., Biochemistry 37 13660-13673 (1998)), der C-terminale Linker des Human Replication Protein A (hsRPA; vgl. D.M. Jacobs et al., J. Biomol. NMR 14, 321-331 (1999)) und der Linker des Parathyroid hormone-related protein (M. Weidler et al. FEBS Lett. 444, 239-244 (1999)). Der Linker hat vorzugsweise eine Länge von bis zu 30 Aminosäuren. Die erfindungsgemäße Nukleinsäuresequenz der obigen Formel enthält daher einen für einen solchen Linker kodierenden Nukleinsäuresequenzabschnitt, vorzugsweise den Hinge von IgG gemäß Nukleotiden 1636 bis 1683 der SEQ ID NO: 1.

Wie bereits erwähnt, kodiert FI für ein Peptid (als gp41-Peptid bezeichnet), das einem Sequenzabschnitt des HIV-gp41-Proteins (vgl. SEQ ID NO: 4, einschließlich der gp41-Proteine der HIV-Quasispezies (clades)) entspricht, der aus einem Bereich ausgewählt ist, der die beiden Heptad-Repeatregionen (vgl. Aminosäurepositionen 539-589 und 622-662 in SEQ ID NO: 3 und 4) einschließt. In diesem Zusammenhang schließt "HIV" alle HIV-Typen ein, insbesondere HIV-1. In diesem Zusammenhang ist beispielsweise zu erwarten, daß es auch bei Verwendung eines HIV-2 gp41-Peptids zu einer Kreuzreaktion mit HIV-1 kommt und umgekehrt.

Die von FI kodierten gp41-Peptide haben vorzugsweise eine Mindestlänge von 28 Aminosäuren, so daß FI mindestens 84 Nukleotide umfaßt. Im Rahmen der vorliegenden Erfindung wird als FI insbesondere eine für die in SEQ ID NO:2 von Position 31 bis 66 dargestellte Aminosäuresequenz (entspricht Peptid T-20) kodierende Nukleinsäuresequenz verwendet, vorzugsweise die in SEQ ID NO:1 von Nukleotid 1528 bis Nukleotid 1635 dargestellte Sequenz. Gemäß einer besonderen Ausführungsorm weist das von FI kodierte Peptid eine Länge von maximal 40 Aminosäuren auf, entsprechend einer Maximallänge von 120 Basen für den kodierenden Bereich FI.

Gemäß einer besonderen Ausführungssform der Erfindung weist die Nukleinsäuresequenz der allgemeinen Formel "SP-FI-Hinge-MSD" die in SEQ ID NO:1 von Nukleotid 1438 bis Nukleotid 1773 dargestellte Sequenz auf, die für das in SEQ ID NO: 2 dargestellte Fusionsprotein kodiert.

Das von den genannten Vektoren bzw. Nukleinsäuresequenzen jeweils kodierte Fusionsprotein hat die allgemeine Formel

$$NH_2\text{- sp - fi - hinge - msd -COOH,}$$

in der

| | |
|---|---|
| "$NH_2$" | das aminoterminale Ende des Proteins bezeichnet, |
| "COOH" | das carboxyterminale Ende des Proteins bezeichnet, |
| "sp" | ein Signalpeptid ist, das den Transfer eines exprimier- ten Peptids in das endoplasmatische Reticulum ermög- licht, |
| "fi" | ein Fragment des gp41-Proteins von HIV ist, das einen Abschnitt aus einer Heptad Repeat-Region enthält, wobei der Abschnitt eine Sequenz von mindestens 28 Aminosäuren zwischen Aminosäurepositionen 539-589 und 622-662 in SEQ ID NO:4 oder Aminosäurepositionen 31-66 in SEQ ID NO:2 aufweist, oder eine Aminosäuresequenz ist, die von der Sequenz dieses Fragments durch Austausch, Deletion oder Insertion einzelner Aminosäuren abweicht, |
| "msd" | ein Transmembrananker eines Typ I-Membranproteins ist und |
| "hinge" | eine Proteinsequenz ist, die als flexibler Linker die Peptide "fi" und "msd" verbindet. |

Bezüglich der Definitionen von "sp", "fi", "hinge" und "msd" wird auf die obigen Definitionen der Sequenzelemente "SP", "FI", "Hinge" und "MSD" verwiesen, wo die strukturellen und funktionellen Merkmale des Fusionsproteins bzw. der einzelnen Protein-Teilbereiche bereits erwähnt sind.

Gemäß einer bevorzugten Ausführungsform kodiert die erfindungsgemäße Nukleinsäure das Fusionsprotein mit der in SEQ ID NO: 2 dargestellten Aminosäuresequenz. Das nach Abspaltung des Signalpeptids daraus erhaltene Fusionsprotein ("fi-hinge-msd") hat demgemäß die in SEQ ID NO: 2 von Position 31 bis Position 111 gezeigte Sequenz.

Erfindungsgemäß eingeschlossen sind ferner Nukleinsäuren, die für Homologe bzw. Varianten und Fragmente des genannten Fusionsproteins kodieren, die die im wesentlichen die gleiche pharmakologische und biologische Wirksamkeit und/oder Immunogenität aufweisen.

Unter Homologen bzw. Varianten werden in diesem Zusammenhang solche Sequenzen verstanden, die von den bislang bekannten natürlichen Sequenzen oder Sequenzabschnitten, durch Austausch Deletion oder Insertion einzelner Aminosäuren abweichen. In diesem Zusammenhang sind insbesondere Homologe, Varianten und Fragmente des in SEQ ID NO: 2 dargestellten Proteins sowie die sie kodierenden Nukleinsäuresequenzen eingeschlossen.

Gegenstand der Erfindung ist ferner ein Vektor, der eine o.g. Nukleinsäuresequenz enthält. Bei dem Vektor handelt es sich vorzugsweise um einen retroviralen Vektor.

Gemäß einer bevorzugten Ausführungsform der Erfindung weist der Vektor den in Figur 1 dargestellten Aufbau auf. Das für das erfindungsgemäße Fusionsprotein kodierende Vektor-Insert der allgemeinen Formel "SP-FI-Hinge-MSD" weist vorzugsweise die in SEQ ID NO: 1 dargestellte Nukleinsäuresequenz auf. Eine Probe des letztgenannten Vektors wurde am 11.11.1999 bei der Deutschen Sammlung von Mikroorganismen und Zellkulturen (DSMZ), Mascheroder Weg 1b, 38124 Braunschweig, Deutschland, unter der Nummer DSM 13139 nach dem Budapester Vertrag hinterlegt.

Der oben genannte Vektors läßt sich zur *in vitro* Transfektion von T-Lymphozyten und hämatopoetischen Stammzellen verwenden, wobei diese transfizierten Zellen HIV-Infizierten zur therapeutischen Behandlung verabreicht werden. Die erfindungsgemäßen Vektoren sind zur Verwendung (d.h. zur unmittelbaren (*in vivo*) Applikation) bei der gentherapeutischen Behandlung von mit HIV Infizierten besonders geeignet, wobei es sich vorzugsweise um Vektoren mit gezieltem Tropismus, d.h. mit Spezifität gegenüber HIV Zielzellen (CD4+ Zellen) handelt. Die Erfindung betrifft daher ferner ein gentherapeutisches Arzneimittel, das einen oben genannten Vektor enthält.

Die vorliegende Erfindung wird nachfolgend anhand von Beispielen näher erläutert.

**<u>Beispiele</u>**

Zellen und Viren

HeLa-, 293- und Phoenix-Ampho-Zellen wurden in Dulbecco's Medium (Gibco, Paisley, Großbritannien), das mit 10% foetalem Kälberserum (fetal calf serum, FCS; Sigma, Deisenhofen, Deutschland) supplementiert war, kultiviert. PM-1 wurde in RPMI mit 10% FCS kultiviert. Die viralen Klone NL4-3/GFP, NL4-3env-GFP und pNL4-3 wurden bereits zuvor beschrieben (NL4-3: Welker R., Harris, M., Cardel, B. & Krausslich, H.G. J. Virol. 72, 8833-8840 (1998); NL4-2env-GFP: He, J., Chen, Y., Farzan, M., et al. Nature 385, 645-649) (1997)). Um den env-defizienten Klon NL4-3env-GFP zu pseudotypisieren, wurden die Plasmide pSNJG, pSVIIIenvJRFL, pSVIIIenvYU2 und pSVIIIenvHXB2 (Welker, R., Harris, M., Cardel, B. & Krausslich, H.G. J. Virol. 72, 8833-8840 (1998); He, J., Chen, Y., Farzan, M., et al. Nature 385, 645-649) (1997); von Laer, D., Thomsen, S., Vogt, B., et al., J. Virol 72, 1424-1430 (1998)) verwendet. Diese Plasmide enthalten die VSV G-cDNA bzw. die cDNA der HIV-Hüllen JR-FL, YU2 und HXB2.

Infektiöse replikationskompetente Viren wurden durch Transfektion von NL4-3 oder NL4-3/GFP- DNA in HeLa-Zellen erzeugt. Pseudotypisierte replikationsinkompetente HIV wurden durch Co-Transfektion von pNL4-3env-GFP und eines der Hüll-Expressionsplasmide in 293-Zellen hergestellt. Die retroviralen Vektoren wurden in Phoenix-Verpackungszellen wie zuvor beschrieben (Grignani F., Kinsella, T., Mencarelli, A., et al., Cancer Res. 58, 14-19 (1998)) verpackt.

Klonierung der retroviralen Vektoren

**M85 und M86:** Die für das Signalpeptid des humanen low affinity nerve growth factor receptor (LNGFR) kodierende Sequenz wurden durch Polymerasekettenreaktion (PCR) ausgehend von dem Vektor dLN unter Verwendung der Primer SPNot+ (Sequenz ID NO:5) und SPBgl2 (SEQ ID NO:6) amplifiziert, wodurch eine NotI-Schnittstelle am 5'-Ende und eine BglII-Schnittstelle am 3'-Ende eingeführt wurden (dLN: Fehse et al., Human Gene Therapy 8 (1997) 1815). Die für das fusionsinhibierende Peptid kodierende Sequenz wurde aus NL4-3 unter Verwendung der Primer T20Bgl+ (M85; SEQ ID NO:7) oder T20Bgl-RGD+ (M86; SEQ ID NO:8) und T20Hind- (SEQ NO ID:9) amplifiziert, wodurch eine BglII-Schnittstelle am 5'-Ende und eine HindIII-Schnittstelle am 3'-Ende eingeführt wurden. Die Fragmente wurden in den mit NotI und HindIII verdauten Vektor pBluescriptKS legiert.

**M87 und M88:** Die Transmembran-Domäne von dLNGFR wurde ausgehend von dLN unter Verwendung eines 5'-Primers, der auch die für die Hinge-Region der murinen IgG schweren Kette und eine BglII-Schnittstelle enthielt (hingeTMBgl+; SEQ ID NO:10), und eines 3'-Primers des retroviralen Vektors dLN (U3-; Sequenz ID NO: 11) amplifiziert. Dieses PCR-Produkt wurde zusammen mit dem Signalpeptid-PCR-Produkt (SPNot+/SPBgl-) in den Vektor pBluescript-KS (nach Verdau mit NotI und HindIII) eingefügt, und die Sequenz für T20 wurde (ausgehend von NL4-3) nachfolgend als PCR-Produkt, das flankierende BglII-Schnittstellen enthielt (durch Verwendung der PCR-Primer T20Bgl+ entsprechend SEQ ID NO: 7 (s.o.) und T20Bgl- entsprechend SEQ ID NO: 12), eingefügt.

**M89 und M90:** Die für T20 kodierende Sequenz mit einem Membran-translokationssignal (mts) wurde ausgehend von NL4-3 unter Verwendung der Primer RGD-T20Not+ (M89; SEQ ID NO: 13) oder T20Not+ (M90; SEQ ID NO: 14) und T20mtsHind- (SEQ ID NO: 15) amplifiziert. Im 5'-Primer ist eine NotI-Schnittstelle vorhanden, und im 3'-Primer liegt eine HindIII-Schnittstelle vor. Das Membran-translokationssignal (mts) wurde mit dem 3'-Primer eingeführt. Das Produkt wurde in den mit NotI und HindIII verdauten Vektor pBluescriptKS eingeführt.

Die für die verschiedenen T20-Fusionsproteine kodierenden Gene wurden dann als NotI $\times$ HindIII-Fragmente zusammen mit der polio-IRES aus SF1 MIN in den Vektor MP1N (Hildinger et al., Hum. Gene Ther. 9 (1998) 33-42) transferiert.

Infektion mit HIV

PM-1-Zellen ($5 \times 10^4$ in 0,5 ml) wurden mit 3000 bis 6000 TCID50 (tissue culture infectious dose 50%) replikationskompetentem HIV infiziert. Für die Analyse der p24-Produktion wurde das Medium am Tag 5 ausgetauscht, die Zellen wurden über Nacht inkubiert, und die zellfreien Überstände wurden unter Verwendung eines p24 ELISA wie zuvor beschrieben (Konvalinka, J., Litterst., M.A., Welker, R., et al. J. Virol. 69, 7180-7186 (1995)) untersucht. Die Ergebnisse sind in Fig. 2 dargestellt.

Die Infektion mit NL4-3/GFP wurde zu den angegebenen Zeitpunkten ferner mittels durchflußzytrometrischer Analyse mit einem FACScalibur (Beckton Dickinson, Heidelberg, Deutschland) überwacht. Dazu wurden PM-1 (mit und ohne die Vektoren MP1N und M85 bis M87) mit einer moi von 0.01 (moi: Infektionsmultiplizität; bezeichnet die Anzahl von Viren-Partikeln, mit denen eine Zelle infiziert wird) mit NL-4/GFP infiziert und an den Tagen 4, 7 und 10 durchflußzytometrisch analysiert. Der Prozentsatz EGFP-positiver und damit HIV-infizierter Zellen ist in Fig. 3 für die verschiedenen Kulturen im Verlauf gezeigt. Die Nachweisgrenze liegt bei ca 0.1 % positiven Zellen.

Untersuchung des Wirkmechanismus

Um zu ermitteln, auf welcher Stufe die HIV-Replikation inhibiert wird, wurden mit dem Klon NL4-3env-GFP "single round"-Infektionen durchgeführt. Der Klon NL4-3env-GFP ist aufgrund einer Mutation im env-Gen replikationsdefekt und exprimiert GFP anstelle von nef. Zur Herstellung infektiöser Virionen wurde der Vektor mit den Hüllen dreier verschiedener HIV-Clone (HXB, YU2 und JR-FL) sowie mit dem G-Protein des vesikulären Stomatitisvirus (VSV G) pseudotypisiert. HXB wird als T-tropisch klassifiziert, mit Verwendung des Co-Rezeptors CXCR4, während YU2 und JR-FL M-tropisch sind und CCR-5 verwenden. Die env-Gene der beiden letztgenannten Klone wurden direkt aus primären HIV-Isolaten kloniert (He, J., Chen, Y., Farzan, M. et al. Nature 385, 645-649 (1997). Diese HIV-Pseudotypen sind zu "single round"-Infektionen in der Lage, verbreiten sich aber nicht über die gesamte Kultur. In PM1/M87 Zellen (d.h. in Zellen, die mit dem erfindungsgemäßen, für das Membran-verankerte T-20-Fusionsprotein kodierenden Vektor transfiziert waren) wurde die Infektion über die drei verschiedenen HIV-Hüllen um einen Faktor von 15 bis 30 stärker inhibiert als im Falle der VSV-G-Pseudotypen, bei denen keine signifikante Inhibierung beobachtet wurde (vergl. Figur 4). Ebenso wie das freie T-20-Peptid (vgl. Wild C.T. et al. Proc. Natl. Acad. Sci. USA 91, 9770-9774 (1994), inhibierte auch das gentechnisch exprimierte Membran-verankerte T-20-Fusionsprotein den durch die Hüllen der verschiedenen HIV-Varianten vermittelten Eintritt. Diese Ergebnisse zeigen eindeutig, daß das Virus auf der Stufe des durch die HIV-Hülle (HIV-env) vermittelten Viruseintritts inhibiert wird, bei der es sich höchstwahrscheinlich um eine Membranfusion handelt. Alle auf den Virus-Eintritt folgenden Schritte der HIV-Replikation wurden nicht beeinflußt.

Diese Untersuchungen zeigen, daß Membran-verankertes gp41-Peptid die HIV-Replikation effizient inhibiert, während sekretiertes reines gp41-Peptid nicht effektiv ist.

## Patentansprüche

1. Nukleinsäure, **gekennzeichnet durch** die Sequenz der allgemeinen Formel

$$5' - SP - FI - hinge - MSD - 3'$$

   in der

   "SP" für ein Signalpeptid kodiert, das den Transfer eines exprimierten Peptids in das endoplasmatische Reticulum ermöglicht,
   "FI" für ein Fragment des gp41-Proteins von HIV kodiert, das einen Abschnitt aus einer Heptad Repeat-Region enthält, wobei der Abschnitt eine Sequenz von mindestens 28 Aminosäuren zwischen Aminosäurepositionen 539-589 und 622-662 in SEQ ID NO:4 oder Aminosäurepositionen 31-66 in SEQ ID NO:2 aufweist,
   "MSD" für einen Transmembrananker eines Typ I- Membranproteins kodiert und
   "Hinge" für eine Proteinsequenz kodiert, die als flexibler Linker die von "FI" und "MSD" kodierten Peptide verbindet.

2. Nukleinsäure nach Anspruch 1, **dadurch gekennzeichnet, dass** SP eine Sequenz ist, die für ein Signalpeptid des low affinity nerve growth factor receptor (LNGFR), des Inter-leukin-2-Rezeptors (IL-2R) oder des granulocyte macrophage colony stimulating factor receptor (GM-CSFR) kodiert.

3. Nukleinsäure nach Anspruch 1 oder 2, **dadurch gekennzeichnet, dass** MSD eine Sequenz ist, die für einen Transmembrananker des LNGFR oder des CD34 oder ein Fragment derselben kodiert.

4. Nukleinsäure nach den Ansprüchen 1 bis 3, **dadurch gekennzeichnet, dass** Hinge eine Sequenz ist, die für den Linker von Immunglobulin G (IgG), des humanen P-Glykoproteins, des Human Replication Proteins A und des Parathyroid hormone-related protein kodiert.

5. Nukleinsäure nach den Ansprüchen 1 bis 4, **dadurch gekennzeichnet, dass** FI für die in SEQ ID NO: 2 von Position 31 bis Position 66 dargestellte Aminosäuresequenz kodiert.

6. Nukleinsäure nach Anspruch 5, **dadurch gekennzeichnet, dass** sie die in SEQ ID NO: 1 von Nukleotid 1528 bis Nukleotid 1635 dargestellte Sequenz aufweist.

7. Nukleinsäure nach den Ansprüchen 1 bis 6, **dadurch gekennzeichnet, dass** sie für ein Protein mit der in SEQ ID NO: 2 dargestellten Sequenz kodiert.

8. Nukleinsäure nach den Ansprüchen 1 bis 7, **dadurch gekennzeichnet, dass** sie die in SEQ ID NO: 1 von Nukleotid 1528 bis Nukleotid 1773 dargestellte Sequenz aufweist.

9. Vektor, **dadurch gekennzeichnet, dass** er eine Nukleinsäure nach den Ansprüchen 1 bis 8 enthält.

10. Vektor nach Anspruch 9, **dadurch gekennzeichnet, dass** er ein retroviraler Vektor ist.

11. Vektor nach Anspruch 10, **dadurch gekennzeichnet, dass** er den in Figur 1 dargestellten Aufbau aufweist.

12. Vektor nach Anspruch 11, **dadurch gekennzeichnet, dass** er die in SEQ ID NO: 1 dargestellte Nukleinsäuresequenz enthält.

13. Vektor nach Anspruch 12 mit der Hinterlegungsnummer DSM 13139.

14. Verwendung eines Vektors nach den Ansprüchen 9 bis 13 zur *in vitro* Transfektion von T-Lymphozyten und hämatopoetischen Stammzellen.

15. Verwendung eines Vektors nach den Ansprüchen 9 bis 13 zur Herstellung einer pharmazeutischen Zusammensetzung zur gentherapeutischen Behandlung von mit HIV Infizierten.

16. Verwendung von T-Lymphozyten oder hämatopoetischen Stammzellen, die mit einem Vektor nach den Ansprüchen 9 bis 13 transfiziert sind, zur Herstellung einer pharmazeutischen Zusammensetzung zur gentherapeutischen Behandlung von mit HIV Infizierten.

17. Gentherapeutisches Arzneimittel, enthaltend einen Vektor nach den Ansprüchen 9 bis 13.

18. Gentherapeutisches Arzneimittel, enthaltend T-Lymphozyten oder hämatopoetischen Stammzellen, die mit einem Vektor nach den Ansprüchen 9 bis 13 transfiziert sind.

## Claims

1. Nucleic acid, **characterised by** the general formula

5'-SP - FI - hinge - MSD-3'

in which

"SP" encodes a signal peptide, which mediates the transfer of an expressed peptide into the endoplasmatic reticulum;
"FI" encodes a fragment of the gp41 protein of HIV, which contains a section from a heptad repeat region of gp41, wherein said section contains a sequence of at least 28 amino acids between amino acid positions 539-589 and 622-662 in SEQ ID NO:4 or between amino acid positions 31-66 in SEQ ID NO:2,

"MSD" encodes a transmembrane-anchor of a type-1 membrane protein; and
"Hinge" encodes a protein sequence, which connects the peptides encoded by "FI" and "MSD" as a flexible linker.

2. Nucleic acid according to Claim 1, **characterised in that** SP is a sequence, which encodes a signal peptide of the low affinity nerve growth factor receptor (LNGFR), of the interleukin-2-receptor (IL-2R) or of the granulocyte macrophage colony stimulating factor receptor (GM-CSFR).

3. Nucleic acid according to Claim 1 or 2, **characterised in that** MSD is a sequence which encodes a trans-membrane anchor of the LNGFR or of the CD34 or a fragment thereof.

4. Nucleic acid according to Claims 1 to 3, **characterised in that** hinge is a sequence which encodes the linker of immunoglobulin G (IgG), of the human P-glycoprotein, of the human replication protein A and of parathyroid hormone-related protein.

5. Nucleic acid according to Claims 1 to 4, **characterised in that** FI encodes the amino acid sequence represented in SEQ ID NO: 2 from position 31 to position 66.

6. Nucleic acid according to Claim 5, **characterised in that** it has the sequence represented in SEQ ID NO: 1 from nucleotide 1528 to nucleotide 1635.

7. Nucleic acid according to Claims 1 to 6, **characterised in that** it encodes a protein having the sequence represented in SEQ ID NO: 2.

8. Nucleic acid according according to Claims 1 to 7, **characterised in that** it has the sequence represented in SEQ ID NO: 1 from nucleotide 1528 to nucleotide 1773.

9. Vector, **characterised in that** it contains a nucleic acid according to Claims 1 to 8.

10. Vector according to Claim 9, **characterised in that** it is a retroviral vector.

11. Vector according to Claim 10, **characterised in that** it has the structure represented in Figure 1.

12. Vector according to Claim 11, **characterised in that** it contains the nucleic acid sequence represented in SEQ ID NO: 1.

13. Vector according to Claim 12 having deposit number DSM 13139.

14. Use of a vector according to Claims 9 to 13 for *in vitro* transfection of T-lymphocytes and haematopoietic stem cells.

15. Use of a vector according to Claims 9 to 13 for the preparation of a pharmaceutical composition for gene therapeutical treatment of patients infected with HIV.

16. Use of T-lymphocytes or haematopoietic stem cells which are transfected with a vector according to Claims 9 to 13 for the preparation of a pharmaceutical composition for gene therapeutical treatment of patients infected with HIV.

17. Gene therapeutical medicament containing a vector according to Claims 9 to 13.

18. Gene therapeutical medicament containing T-lymphocytes or haematopoietic stem cells which are transfected with a vector according to Claims 9 to 13.

## Revendications

1. Acide nucléique, **caractérisé par** la séquence de formule générale :

$$5' - SP - FI - hinge - MSD - 3'$$

dans laquelle

"SP" code pour un peptide signal qui permet le transfert dans le réticulum endoplasmique d'un peptide exprimé,
"FI" code pour un fragment de la protéine gp41 du virus HIV, qui contient un segment d'une heptad repeat region, le segment présentant une séquence d'au moins 28 acides aminés entre les positions d'acides aminés 539-589 et 622-662 dans SEQ ID NO:4 ou les positions d'acides aminés 31-66 dans SEQ ID NO:2,
"MSD" code pour une ancre transmembranaire d'une protéine de membrane du type I,
"hinge" code pour une séquence de protéine qui lie en tant que linker flexible les peptides codés par "FI" et "MSD".

2. Acide nucléique suivant la revendication 1, **caractérisé en ce que** SP est une séquence qui code pour un peptide signal du low affinity nerve growth factor receptor (LNGFR), du récepteur d'interleukine-2 (IL-2R) ou du granulocyte macrophage colony stimulating factor receptor (GM-CSFR).

3. Acide nucléique suivant la revendication 1 ou 2, **caractérisé en ce que** MSD est une séquence qui code pour une ancre transmembranaire du LNGFR ou de CD34 ou un de leurs fragments.

4. Acide nucléique suivant les revendications 1 à 3, **caractérisé en ce que** hinge est une séquence qui code pour le linker de l'immunoglobuline G (IgG), de la P-glycoprotéine humaine, de human replication protein A et de parathyroid hormone-related protein.

5. Acide nucléique suivant les revendications 1 à 4, **caractérisé en ce que** FI code pour la séquence d'acides aminés représentée de la position 31 à la position 66 dans SEQ ID NO: 2.

6. Acide nucléique suivant la revendication 5, **caractérisé en ce qu'**il présente la séquence représentée du nucléotide 1528 au nucléotide 1635 dans SEQ ID NO: 1.

7. Acide nucléique suivant les revendications 1 à 6, **caractérisé en ce qu'**il code pour une protéine ayant la séquence représentée dans SEQ ID NO: 2.

8. Acide nucléique suivant les revendications 1 à 7, **caractérisé en ce qu'**il présente la séquence représentée du nucléotide 1528 au nucléotide 1773 dans SEQ ID NO: 1.

9. Vecteur, **caractérisé en ce qu'**il contient un acide nucléique suivant les revendications 1 à 8.

10. Vecteur suivant la revendication 9, **caractérisé en ce qu'**il est un vecteur rétroviral.

11. Vecteur suivant la revendication 10, **caractérisé en ce qu'**il présente la structure représentée sur la figure 1.

12. Vecteur suivant la revendication 11, **caractérisé en ce qu'**il contient la séquence d'acide nucléique représentée dans SEQ ID NO: 1

13. Vecteur suivant la revendication 12 affecté du numéro de dépôt DSM 13139.

14. Utilisation d'un vecteur suivant les revendications 9 à 13 pour la transfection *in vitro* de lymphocytes T et de cellules souches hématopoïétiques.

15. Utilisation d'un vecteur suivant les revendications 9 à 13 pour la préparation d'une composition pharmaceutique destinée au traitement par thérapie génique de patients infectés par le virus HIV.

16. Utilisation de lymphocytes T ou de cellules-souches hématopoïétiques, qui ont été transfectés avec un vecteur suivant les revendications 9 à 13, pour la préparation d'une composition pharmaceutique destinée au traitement par thérapie génique de patients infectés par le virus HIV.

17. Médicament destiné à la thérapie génique, contenant un vecteur suivant les revendications 9 à 13.

18. Médicament destiné à la thérapie génique, contenant des lymphocytes T ou des cellules souches hématopoïétiques qui ont été transfectés avec un vecteur suivant les revendications 9 à 13.

Fig. 1 A:

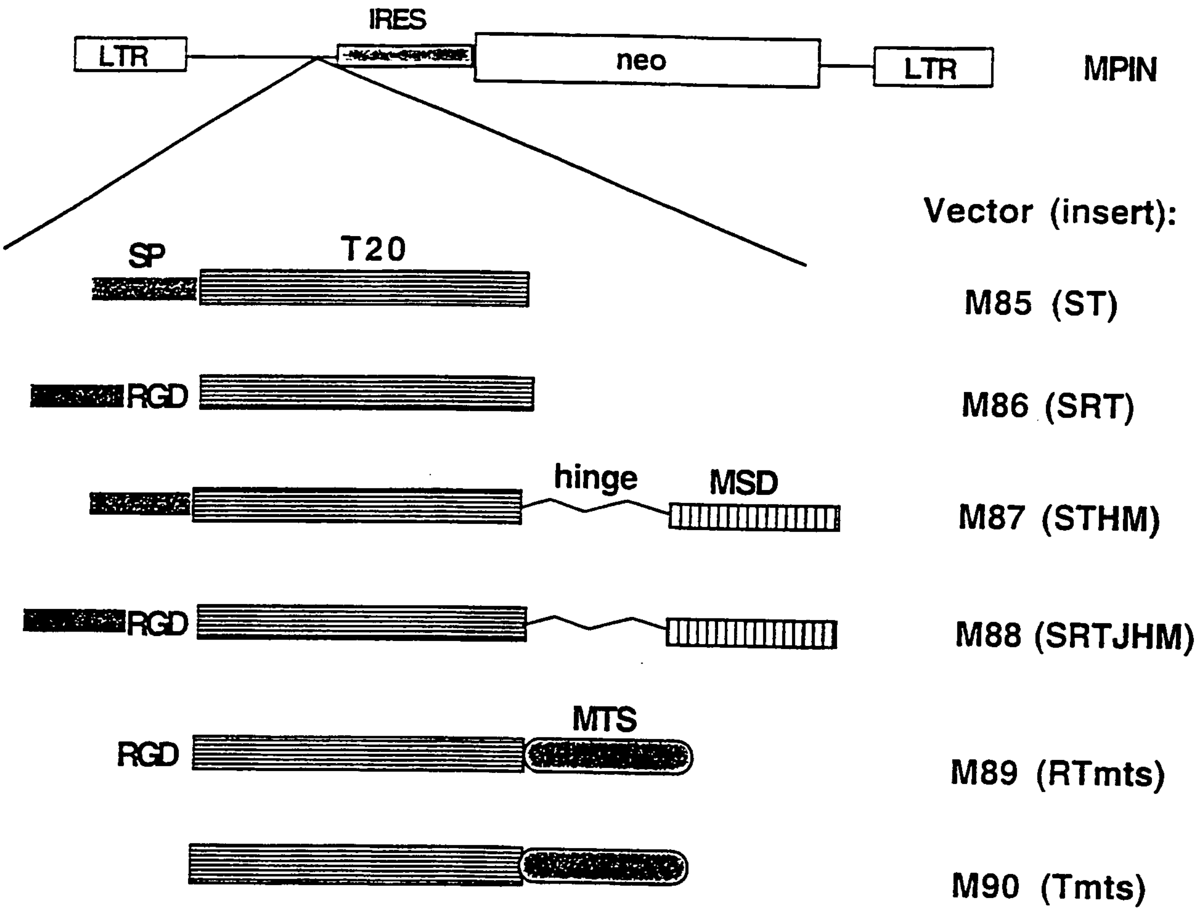
IRES
LTR
neo
LTR
MPIN
Vector (insert):
SP
T20
M85 (ST)
RGD
M86 (SRT)
hinge
MSD
M87 (STHM)
RGD
M88 (SRTJHM)
MTS
RGD
M89 (RTmts)
M90 (Tmts)

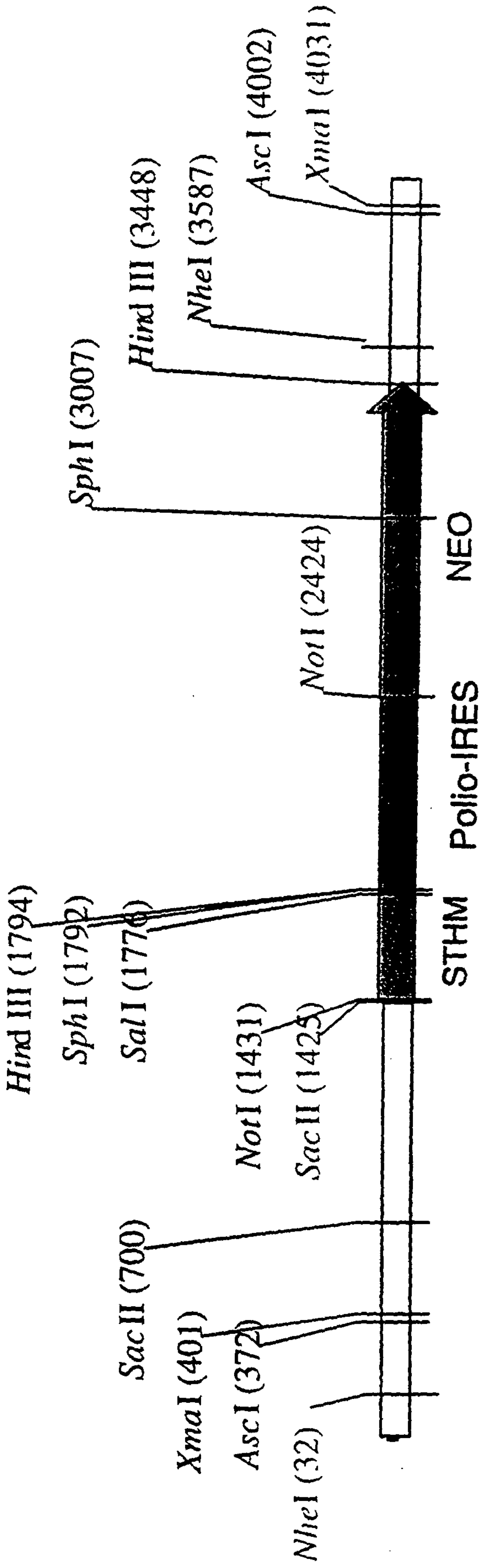


Fig. 1 B: **MP1 STHM IRES NEO**
4148 bp

Fig. 2: **Nachweis der HIV-Production in Lymphozyten mit verschiedenen antiviralen Vektoren am Tag 6**

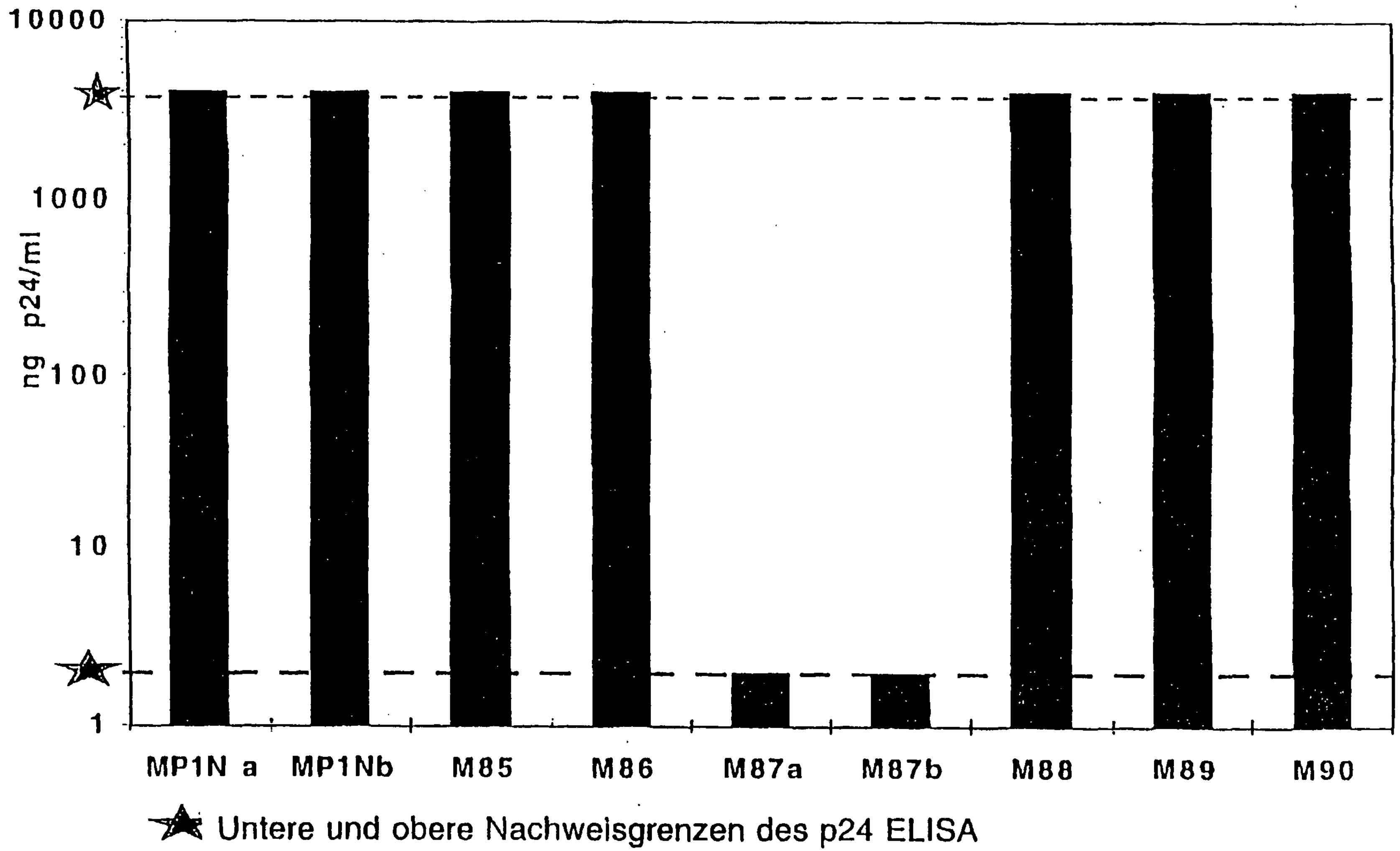

Fig. 3:

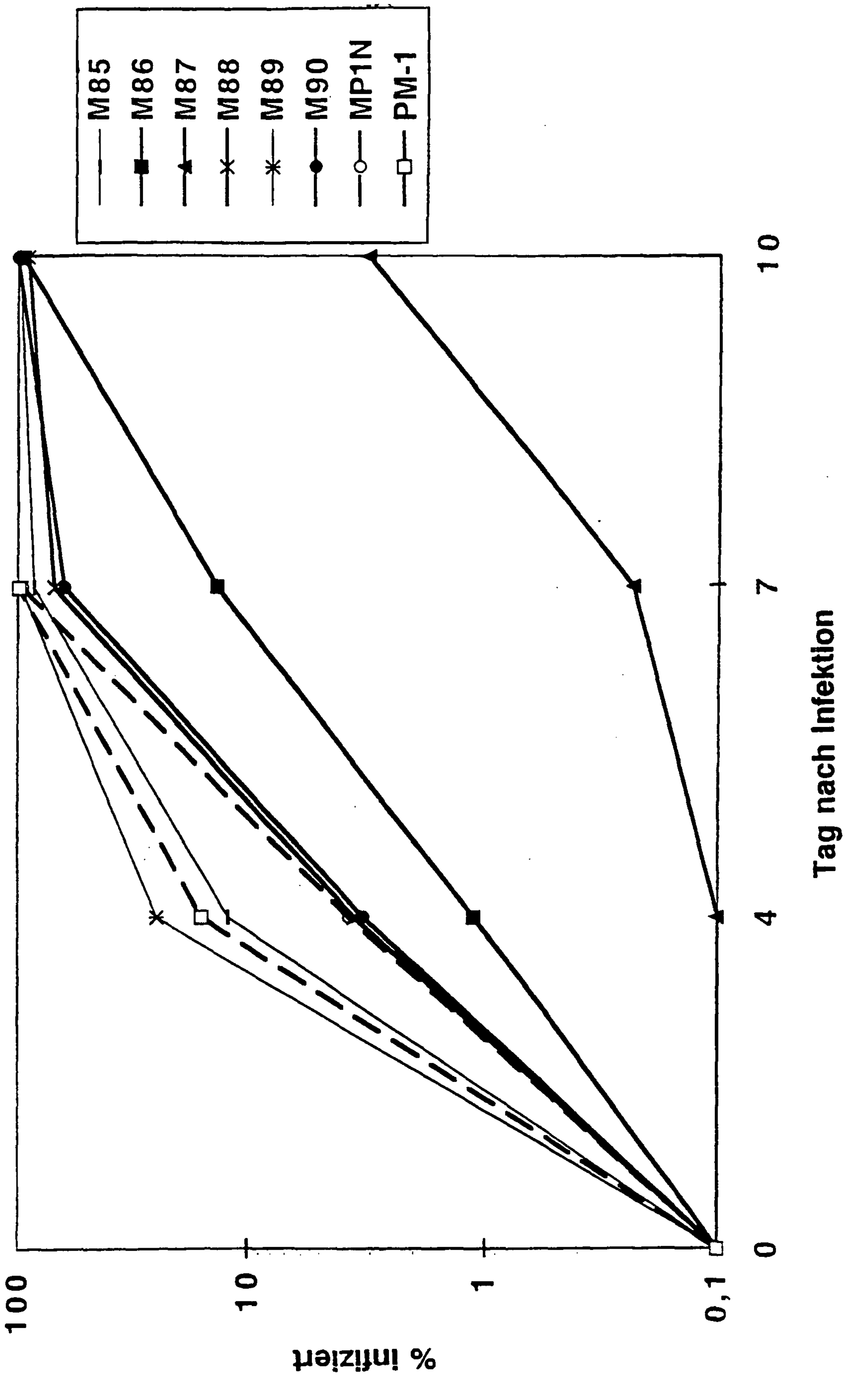
M85
M86
M87
M88
M89
M90
MP1N
PM-1
100
10
1
0,1
% infiziert
0
4
7
10
Tag nach Infektion

Fig. 4:

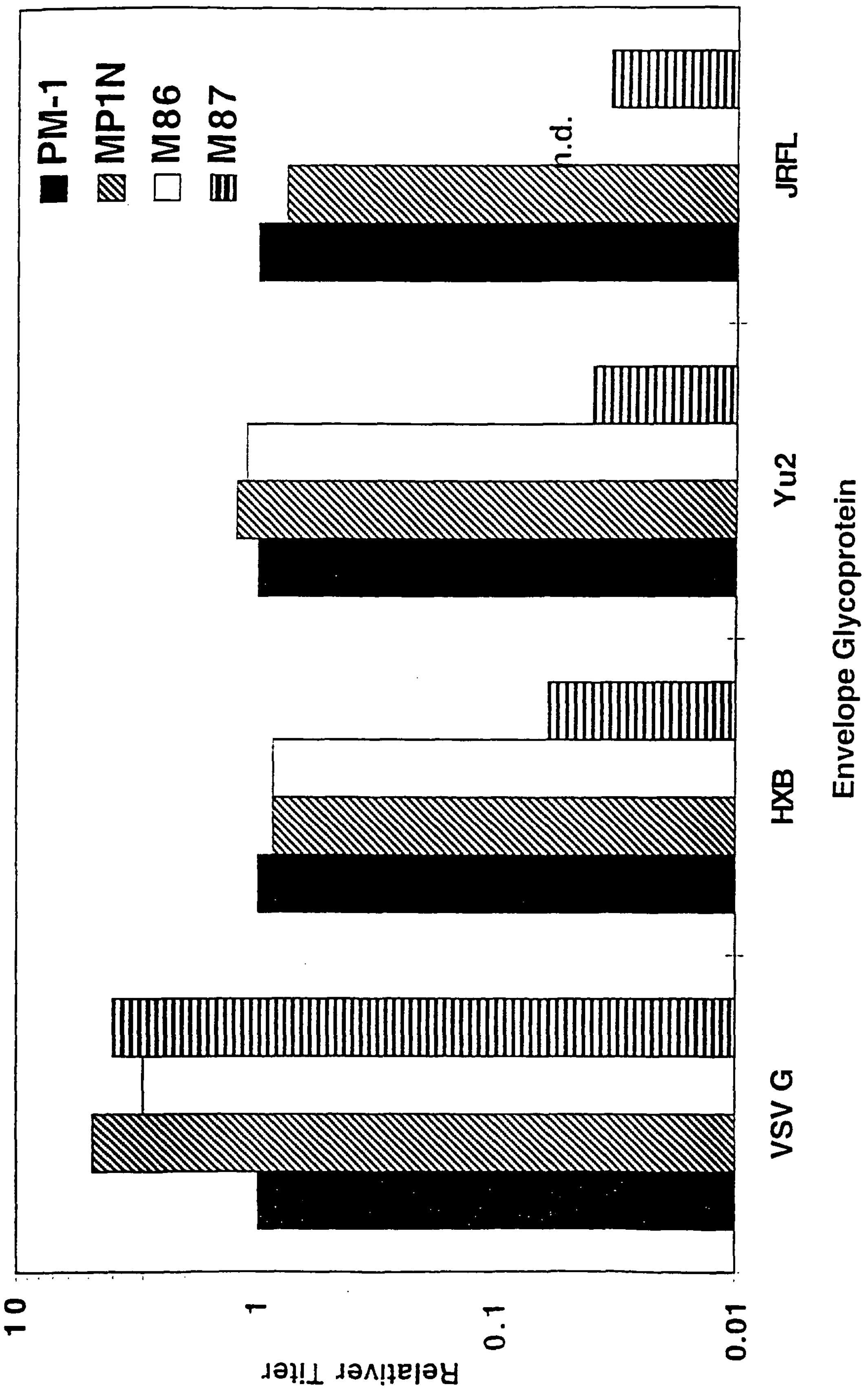
PM-1
MP1N
M86
M87
n.d.
10
1
0.1
0.01
Relativer Titer
VSV G
HXB
Yu2
JRFL
Envelope Glycoprotein

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **SORG T. ; METHALI, M.** *Transfus. Sci.,* 1997, vol. 18, 277-289
- **WILD, C.T. ; SHUGARS, D.C. ; GREENWELL, T.K. ; MCDANAL, C.B. ; MATTHEWS, T.J.** *Proc. Natl.Acad. Sci U.S.A.,* 1994, vol. 91, 9770-9774
- **WEISSENHBORN, W. ; DESSEN, A. ; HARRISON, S.C. ; SKEHEL, J.J. ; WILEY, D.C.** *Nature,* 1997, vol. 387, 426-430
- **CHAN, D.C. ; FASS, D. ; BERGER, J.M. ; KIM, P.S.** *Cell,* 1997, vol. 89, 263-273
- **FURUTA, R.A. ; WILD, C.T. ; WENG Y ; WEISS, C.D.** *Nat. Struct. Biol.,* 1998, vol. 5, 276-279
- **KILBY, J.M. ; HOPKINS, S. ; VENETTA, T.M. et al.** *Nat.Med.,* 1998, vol. 4, 1302-1307
- **HILDINGER et al.** *Hum. Gene Ther.,* 1998, vol. 9, 33-42
- **FEHSE et al.** *Human Gene Therapy,* 1997, vol. 8, 1815
- **ROJAS, M. ; DONAHUE, J.P. ; TAN, Z. ; LIN, Y.Z.** *Nat.Biotechnol.,* 1998, vol. 16, 370-375
- **GRIGNANI, F. ; KINSELLA, T. ; MENCARELLI, A. et al.** *Cancer Res.,* 1998, vol. 58, 14-19
- **BOU-HABIB, D.C. et al.** *J. Virol.,* 1994, vol. 68, 6006-6013
- **ADACHI, A. ; GENDELMAN H.E. ; KOENIG, S. ; FOLKS, T. ; WILLEY, R. ; RABSON, A. ; MARTIN, M.A.** *J. Virol.,* 1986, vol. 59, 284-291
- **WELKER, R. ; HARRIS, M. ; CARDEL, B. ; KRAUSSLICH, H.G.** *J.Virol.,* 1998, vol. 72, 8833-8840
- **C.A. HRYCYNA et al.** *Biochemistry,* 1998, vol. 37, 13660-13673
- **D.M. JACOBS et al.** *J. Biomol. NMR,* 1999, vol. 14, 321-331
- **M. WEIDLER et al.** *FEBS Lett.,* 1999, vol. 444, 239-244
- **WELKER R. ; HARRIS, M. ; CARDEL, B. ; KRAUSSLICH, H.G.** *J. Virol.,* 1998, vol. 72, 8833-8840
- **HE, J. ; CHEN, Y. ; FARZAN, M. et al.** *Nature,* 1997, vol. 385, 645-649
- **WELKER, R. ; HARRIS, M. ; CARDEL, B. ; KRAUSSLICH, H.G.** *J. Virol.,* 1998, vol. 72, 8833-8840
- **VON LAER, D. ; THOMSEN, S. ; VOGT, B. et al.** *J. Virol,* 1998, vol. 72, 1424-1430
- **GRIGNANI F. ; KINSELLA, T. ; MENCARELLI, A. et al.** *Cancer Res,* 1998, vol. 58, 14-19
- **KONVALINKA, J. ; LITTERST., M.A. ; WELKER, R. et al.** *J. Virol.,* 1995, vol. 69, 7180-7186
- **WILD C.T. et al.** *Proc. Natl. Acad. Sci. USA,* 1994, vol. 91, 9770-9774